# EUROPEAN PATENT SPECIFICATION

(11) **EP 1 929 960 B1**
(45) Date of publication and mention of the grant of the patent: **11.11.2009**
(21) Application number: 07017969.2
(22) Date of filing: 13.09.2007
(51) Int. Cl.: A61B 17/04, A61B 17/06

(54) **Perforation suture clip and clip device**
Perforationsnahtklemme und Klemmvorrichtung
Pince de suture à perforation et dispositif de pince

(30) Priority: 14.11.2006 JP 2006307638
(43) Date of publication of application: 11.06.2008
(73) Proprietor: OLYMPUS MEDICAL SYSTEMS CORP., Shibuya-ku Tokyo 151-0072 (JP)
(72) Inventor: Matsushita, Masumi, Shibuya-ku Tokyo (JP)
(74) Representative: von Hellfeld, Axel

(56) References cited:
- WO-A-96/03083
- US-A- 5 059 207
- US-A1- 2004 078 054

## Description

### BACKGROUND OF THE INVENTION

### Field of the Invention

The present invention relates to a perforation suture clip that is used in combination with an endoscope in order to suture a perforation that has occurred in a digestive tract, and to a clip device that is provided with this perforation suture clip.

Priority is claimed on Japanese Patent Application No. 2006-307638 , filed November 14, 2006.

### Description of Related Art

Generally, if a perforation occurs for whatever reason in a digestive tract such as in an esophagus or a stomach, an abdominal operation is performed and the perforation is closed. However, in this method, because the abdomen is always cut open irrespective of the size of the perforation, the burden on the patient is considerable and post-operation recovery takes a long time.

Therefore, several manual methods of closing a perforation without performing an abdominal operation have been proposed. A toggle that is used in combination with a needle is known as one such method (see, for example, Patent Document 1: United States Patent (USP) No. 6,319,263 B1). This toggle has a thread fixed to it and is housed inside a needle that is made to puncture a body from the exterior to the interior using a hard endoscope or the like. As a result, the toggle is able to penetrate the interior of the digestive tract from outside the body. A portion of the toggle protrudes from the needle, and when the needle is extracted from the digestive tract, this protruding portion becomes caught and is left behind in the digestive tract.

When a perforation is closed using this toggle, a needle is made to puncture the digestive tract surrounding the perforation and the toggle is made to penetrate the interior of the digestive tract. Next, the needle is extracted so that the toggle is left behind in the digestive tract. At this time, the thread that has been fixed to the toggle is withdrawn to the outside of the digestive tract. In this manner, at least two toggles are left inside the digestive tract around the perforation. Next, the threads that are fixed to each toggle are firmly secured and the toggles are pulled towards each other. When the toggles are pulled together, because the biological tissue surrounding the perforation is pulled together as a consequence of being pulled by the toggles, the perforation can be closed.

In this manner, according to a method in which toggles are utilized, because a perforation can be closed without an abdominal operation being performed, it is possible to reduce the burden on the patient.

However, in the method which utilizes toggles described in Patent Document 1, the following problems still remain.

Namely, although it is possible to close a perforation by pulling the toggles after securing the threads that are fixed to the toggles, because the threads are only secured outside the digestive tract, it is only possible to reef the portion on the serous membrane side (i.e., the anchor side) of the digestive tract. Accordingly, it has not been possible to reef the mucous membrane side (i.e., the inner side) of the digestive tract in the same way as the serous membrane side so that, when viewed from the mucous membrane side, the location of the perforation remains in an open state. Because of this, it has not been possible to reliably suture a perforation from both the serous membrane side and the mucous membrane side so that there are defects in the reliability of the suturing. Moreover, in order to puncture percutaneously to the interior of a body and reach a perforation in a digestive tract, it has been necessary to form a hole in the abdominal wall.

### SUMMARY OF THE INVENTION

The present invention was conceived in view of the above described problem points in the conventional technology, and it is an object thereof to provide a perforation suture clip and a clip device that is equipped with this perforation suture clip that do not require an abdominal operation and do not require a hole to be formed in an abdominal wall, and that make it possible to reef a perforation occurring in a digestive tract from both a serous membrane side and a mucous membrane side so that suturing can be performed more reliably.

In order to achieve the above described object, this invention provides the following means.

The present invention is a perforation suture clip that is used in combination with an endoscope and sutures a perforation occurring in a digestive tract, characterized in that: the perforation suture clip includes a rod portion whose two ends are sharpened; a hook portion that is bent back from one end side of the rod portion at a predetermined opening angle towards the other end side of the rod portion; and a traction portion that is fixed to the other end side of the rod portion and drags the other end side of the rod portion.

In the perforation suture clip of the present invention, firstly, using an endoscope, a rod portion punctures a digestive tract adjacent to a perforation from the interior of the digestive tract. At this time, because both ends of the rod portion have been sharpened, it is possible for the other end side of the rod portion to penetrate from the mucous membrane side (i.e., from the inner side of the digestive tract), or for the rod portion to be moved first to the outside of the digestive tract through the perforation, and for the one end side thereof to then penetrate from the serous membrane side (i.e., from the outer side of the digestive tract). In either case, as a result of this puncture, one end side of the rod portion can be positioned on the mucous membrane side while the other end side of the rod portion can be positioned on the serous membrane side. Namely, the hook portion can be positioned on the mucous membrane side which is the inside of the digestive tract, while the traction portion can be positioned on the serous membrane side which is the outside of the digestive tract. In this way, a plurality of (i.e., at least two) rod portions puncture the digestive tract in the vicinity of a perforation.

Next, the other end side of the rod portion is dragged via the traction portion so as to be pulled to the digestive tract side. As a result, the serous membrane side of the digestive tract can be gradually pulled, and the serous membrane side of the perforation can be closed off while in a reefed state. Moreover, at this time, the one end sides of the plurality of rod portions remain positioned on the mucous membrane side. Accordingly, by joining together the one end sides of the plurality of rod portions using a wire material or the like while they are positioned adjacent to each other, the mucous membrane sides of the digestive tract can be pulled in the same way. Accordingly, the mucous membrane side of the perforation can also be closed off while in a reefed state. In particular, because the hook portion is formed at the one end side of the rod portion, the wire material does not drop off the rod portion.

As is described above, according to the perforation suture clip of the present invention, it is possible to reef a perforation occurring in a digestive tract from both a serous membrane side and a mucous membrane side and perform reliable suturing without performing an abdominal operation and without opening a hole in a stomach wall. In particular, unlike a conventional device, because it is possible to close the perforation on both the inner side and outer side of the digestive tract, the suturing is secure and the reliability of an operation can be improved.

In the perforation suture clip according to the present invention, it is also possible for the traction portion to be a folded-back portion that is folded back at a predetermined opening angle towards the one end side of the rod portion, and is drawn into the perforation.

In the perforation suture clip according to the present invention, because the traction portion is folded back, during the puncturing by the rod portion, this folded-back portion can be hooked onto the digestive tract so that the rod portion can be easily placed in a predetermined position. Moreover, because the folded-back portion is drawn into the perforation and is hooked onto the digestive tract, when external force is applied this force can be transmitted to the digestive tract. Because of this, the other end side of the rod portion can be easily and reliably pulled to the digestive tract side. Accordingly, the serous membrane side of the digestive tract can be more reliably reefed and closed up.

In the perforation suture clip according to the present invention, it is also possible for the traction portion to be a traction thread that has a predetermined tensile strength, and is drawn through the perforation to a mucous membrane side.

In the perforation suture clip according to the present invention, because external force can be applied easily simply by pulling a traction thread that has been drawn through the perforation to the mucous membrane side, the task of reefing the serous membrane side can be performed easily. Moreover, because the traction thread is drawn to the mucous membrane side, there is an improvement in workability.

The present invention is a clip device that is used in combination with an endoscope and sutures a perforation occurring in a digestive tract, characterized in that: the clip device includes the above described perforation suture clip; a housing tool that is inserted into a channel of the endoscope, and whose interior houses the rod portion with the other end side facing towards a distal end of the housing tool; an engaging portion that engages with the hook portion; an operation transmitting device that is connected to the engaging portion, and is able to move backwards and forwards freely relative to the housing tool; and an operating section that is connected to the operation transmitting device, and moves the housed rod portion backwards and forwards in the axial direction of the housing tool by moving the operation transmitting device backwards and forwards, and that terminates the engagement between the engaging portion and the hook portion by pushing the rod portion out from the housing tool.

In the clip device according to the present invention, because the perforation suture clip can be housed within a housing tool, the perforation suture clip can approach the vicinity of the perforation without being brought into contact with any other portions. Accordingly, it is possible to further improve the level of safety of an operation. Moreover, by moving the operation transmitting device forwards or backwards using the operating section, the perforation suture clip that is engaged with the engaging portion can be pushed out from the housing tool and made to puncture the digestive tract. Moreover, the rod portion can be pushed out from the housing tool at a desired timing so that the engagement thereof with the hook portion can be disengaged, and the perforation suture clip can be easily detached and left in the digestive tract. In this manner, the perforation suture clip can be handled easily and an improvement in operability can be achieved.

In the clip device according to the present invention, it is also possible for a plurality of the perforation suture clips to be housed in the housing tool by being lined up in a row in the axial direction.

In the clip device according to the present invention, because a plurality of perforation suture clips are arranged in a row and housed within the housing tool, it is possible for a rod portion to puncture a digestive tract adjacent to a perforation efficiently and quickly. Accordingly, an improvement in workability can be achieved and work time can be shortened.

In the clip device according to the present invention, it is also possible for the distal end of the housing tool to be formed in a needle shape that enables the housing tool to puncture the digestive tract.

In the clip device according to the present invention, because the distal end of the housing tool is formed in a needle shape, the housing tool itself is able to puncture the digestive tract. Thereafter, by pushing the perforation suture clip out from inside the housing tool via the operating section and extracting the housing tool, the perforation suture clip can be easily positioned in a predetermined location.

In this manner, because it is not necessary for the perforation suture clip to directly puncture the digestive tract, even if the muscular tunic between the mucous membrane side and the serous membrane side is comparatively strong, it is still possible to reliably leave the perforation suture clip in a predetermined position.

According to the perforation suture clip of the present invention, it is possible to reef a perforation occurring in a digestive tract from both a serous membrane side and a mucous membrane side and perform reliable suturing without performing an abdominal operation and without opening a hole in a stomach wall. Accordingly, the reliability of an operation can be improved.

Moreover, according to the clip device of the present invention, the perforation suture clip can be handled easily and an improvement in operability can be achieved.

### BRIEF DESCRIPTION OF THE DRAWINGS

FIG 1 is a view showing a first embodiment according to the present invention, and is a view showing a state in which a housing sheath that houses a perforation suture clip is inserted inside a treatment tool channel of an endoscope.
FIG 2 is a cross-sectional view showing an enlargement of an end portion of the housing sheath shown in FIG. 1.
FIG 3 is a plan view of a perforation suture clip that is housed in the housing sheath shown in FIG. 2.
FIG 4 is a view showing a state in which, from the state shown in FIG 2, the perforation suture clip is pushed from the housing sheath.
FIG 5 is a view showing each step when a perforation occurring in a stomach is sutured using the clip device shown in FIG 1, and shows a state in which a needle portion at a distal end of a housing sheath has punctured a stomach wall in the vicinity of a perforation.
FIG 6 is a view showing a state in which, from the state shown in FIG. 5, the housing sheath is pushed forward so as to penetrate the stomach wall.
FIG 7 is a view showing a state in which, from the state shown in FIG 6, the other end side of a rod portion is pushed out from the distal end of the housing sheath.
FIG 8 is a view showing a state in which, from the state shown in FIG 7, the other end side of the rod portion is made to face upwards towards a perforation side by pulling the entire housing sheath into the stomach interior.
FIG 9 is a view showing a state in which, from the state shown in FIG 8, a traction fiber is drawn through the perforation into the interior of the stomach.
FIG 10 is a view showing a state in which, from the state shown in FIG 9, a perforation suture clip is pulled into the interior of the stomach, and a gap is opened between a hook portion and the stomach wall.
FIG 11 is a view showing a state in which, from the state shown in FIG 10, the perforation suture clip is left remaining in the stomach wall.
FIG 12 is a view showing a state in which, from the state shown in FIG 11, another perforation suture clip is left remaining in a position that sandwiches the perforation.
FIG 13 is a view showing a state in which, from the state shown in FIG. 12, the stomach wall is pulled shut by pulling the traction threads of the respective perforation suture clips so as to reef the serous membrane side.
FIG 14 is a view showing a state in which, from the state shown in FIG 13, a loop is hooked onto one end side of rod portions of the respective perforation suture clips.
FIG. 15 is a view showing a state in which, from the state shown in FIG 14, the mucous membrane side is reefed by pulling tight the loop so that the one end sides of the rod portions are joined together in a state of contact with each other, resulting in the perforation becoming completely closed.
FIG. 16 is a view showing a second embodiment according to the present invention, and is a cross-sectional view of a housing sheath that houses a perforation suture clip in which a folded-back portion is formed.
FIG 17 is a view showing each step when a perforation occurring in a stomach is sutured using the perforation suture clip shown in FIG. 16, and shows a state in which a needle portion at a distal end of a housing sheath has punctured a stomach wall in the vicinity of a perforation.
FIG 18 is a view showing a state in which, from the state shown in FIG 17, the housing sheath is pushed forward so as to penetrate the stomach wall.
FIG 19 is a view showing a state in which, from the state shown in FIG 18, the folded-back portion that is formed at the other end side of the rod portion is pushed out from the distal end of the housing sheath.
FIG 20 is a view showing a state in which, from the state shown in FIG 19, the entire housing sheath is pulled into the stomach interior and the folded-back portion becomes caught on the stomach wall so as to be drawn into the stomach interior.
FIG 21 is a view showing a state in which, from the state shown in FIG 20, a perforation suture clip is pulled into the interior of the stomach, and a gap is opened between a hook portion and the stomach wall.
FIG 22 is a view showing a state in which, from the state shown in FIG 21, the perforation suture clip is left remaining in the stomach wall, and another perforation suture clip is also left remaining in a position that sandwiches the perforation.
FIG. 23 is a view showing a state in which, from the state shown in FIG 22, the stomach wall is pulled shut via the folded-back portion by pulling the respective perforation suture clips so as to reef the serous membrane side.
FIG 24 is a view showing a state in which, from the state shown in FIG 23, a loop is hooked onto one end side of rod portions of the respective perforation suture clips.
FIG 25 is a view showing a state in which, from the state shown in FIG 24, the mucous membrane side is reefed by pulling tight the loop so that the one end sides of the rod portions are joined together in a state of contact with each other, resulting in the perforation becoming completely closed.
FIG. 26 is a view showing another example of the clip device according to the present invention, and shows a state in which a perforation suture clip is housed within a housing sheath having a circular cylinder-shaped distal end.

### DETAILED DESCRIPTION OF THE INVENTION

### (First embodiment)

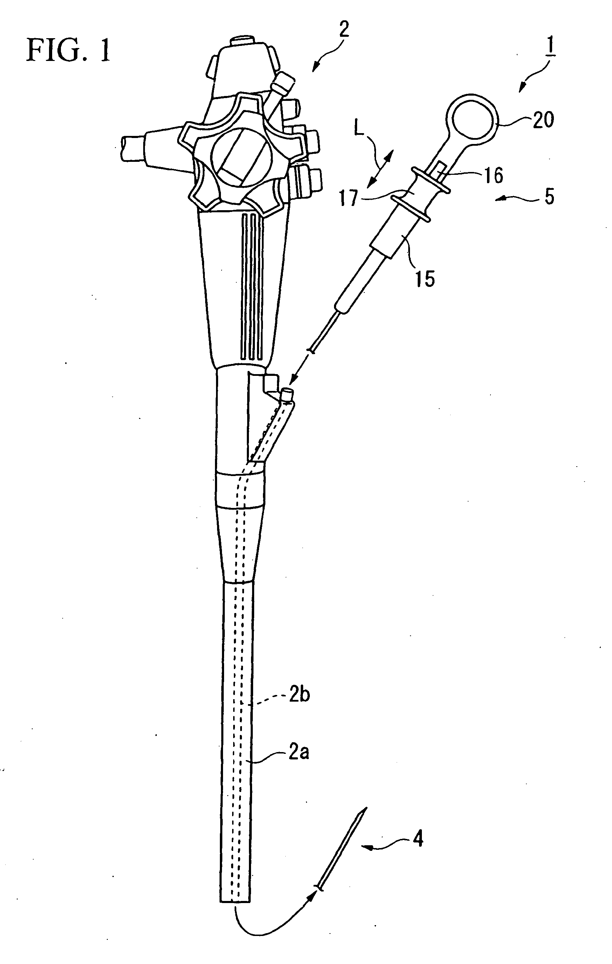

The first embodiment of the perforation suture clip and clip device according to the present invention will now be described with reference made to FIGS. 1 through 15. Note that in the present embodiment a case in which a perforation H occurs in a stomach is described as an example of a perforation H occurring in a digestive tract.

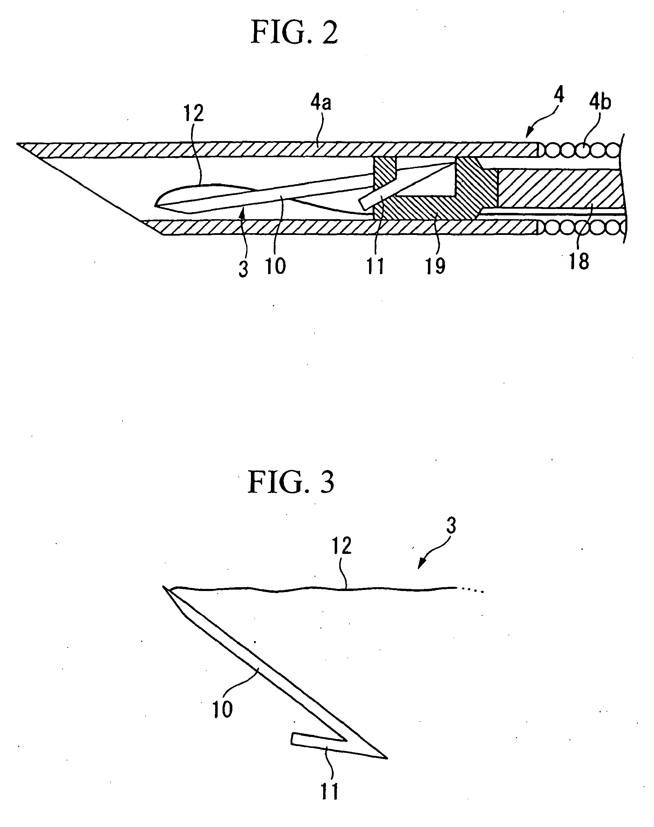

As is shown in FIG 1, the clip device 1 of the present embodiment is used in combination with an endoscope 2. As is shown in FIGS. 1 and 2, this clip device 1 is provided with a perforation suture clip 3 that sutures the perforation H, a housing sheath (i.e., a housing tool) 4 that is inserted inside a treatment tool channel 2b that is formed in an insertion portion 2a of the endoscope 2 and whose interior houses the perforation suture clip 3, and an operating section 5 that operates the perforation suture clip 3.

As is shown in FIG 3, the perforation suture clip 3 is formed by a rod portion 10 that has two sharpened ends and punctures a stomach wall X with one end side being located on a mucous membrane side P1 of a stomach and the other end side being located on a serous membrane side P2 of the stomach, a hook portion 11 that is folded back from one end side of the rod portion 10 to the other end side of the rod portion 10 so as to hold a predetermined opening angle, and a traction thread (i.e., a traction portion) 12 that is fixed to the other end side of the rod portion 10 and pulls the other end side towards the stomach wall X side when external force is applied thereto. Note that the rod portion 10 and the hook portion 11 are formed from a metal material such as stainless steel or a titanium alloy, or from a material that does not affect the human body.

The rod portion 10 may be formed having a circular cross section, or it may be formed having a rectangular cross section. The cross-sectional configuration may also be designed freely in accordance with the location and the like of the perforation H. The length of the rod portion 10 may also be designed freely in the same way. The hook portion 11 is folded back by a length that allows it to catch onto a loop (i.e., wire material) 22 (described below) to be caught.

The traction thread 12 is formed from a material that does not affect the human body, and has a predetermined tensile strength.

Moreover, the length of the traction thread 12 is adjusted such that it is longer than at least the length of the rod portion 10. After the rod portion 10 has punctured the stomach wall X, the traction thread 12 is drawn to the mucous membrane side P1, which is the stomach interior, through the perforation H. This operation is described below in detail.

As is shown in FIG. 2, the perforation suture clip 3 that is structured in this manner is housed within a needle portion 4a of the aforementioned housing sheath 4 with the other end side to which the traction thread 12 is fixed facing towards the distal end.

This housing sheath 4 is formed by a coil sheath portion 4b that is made from steel wire, and the needle portion 4a that is fixed to the distal end of the coil sheath portion 4b. The needle portion 4a is formed having a sharpened needle-shaped distal end so as to enable it to puncture the stomach wall X. Namely, the housing sheath 4 of the present embodiment is formed such that the stomach wall X is able to be punctured by the actual housing sheath 4 itself.

As is shown in FIG 1, a base end side of the coil sheath 4b is connected to an operating section main body 15. A guide groove 16 is formed extending in an axial direction L in an intermediate portion of the operating section main body 15. In addition, a sliding portion 17 that slides along the guide groove 16 is fitted to the operating section main body 15.

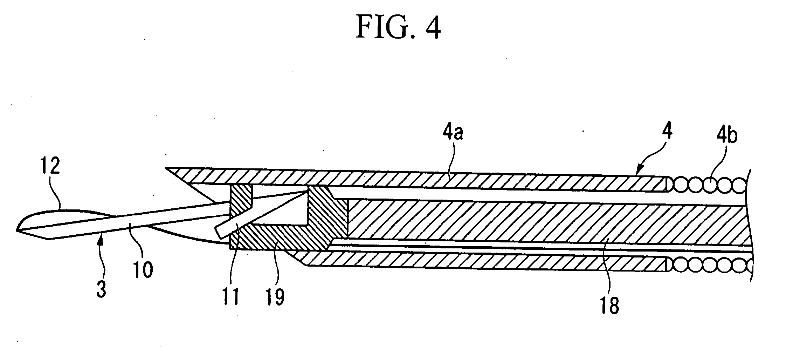

A base end side of a wire (i.e., an operation transmitting device) 18 shown in FIG 2 that is inserted such that it can move freely forwards and backwards through the coil sheath 4b and the needle portion 4a is fixed to the sliding portion 17. A delivery hook (i.e., an engaging portion) 19 that is able to engage with the hook portion 11 of the housed rod portion 10 is fixed to a distal end side of this wire 18. Accordingly, when the sliding portion 17 is moved backwards or forwards, as is shown in FIG 4, it is possible to move the delivery hook 19 towards the distal end of the needle portion 4a or, as is shown in FIG 2, move the delivery hook 19 towards the base end side of the needle portion 4a. As a result, it is possible to push the perforation suture clip 3 out from the housing sheath 4 or pull it into the housing sheath 4, and it is also possible after the clip 3 has been pushed out to release the engagement between the delivery hook 19 and the hook portion 11 and separate the two.

Namely, the operating section main body 15 and the slider portion function as the aforementioned operating section 5 that causes the housed rod portion 10 to move forwards or backwards in the axial direction L of the housing sheath 4 as a result of the wire 18 being moved forwards or backwards, and that also causes the engagement between the delivery hook 19 and the hook portion 11 to be released at a desired timing as a result of the rod portion 10 being pushed out from the housing sheath 4.

Note that, as is shown in FIG 1, a finger ring 20 through which a thumb can be inserted is attached to the base end side of the operating section main body 15. Moreover, the outer circumferential surface of the sliding portion 17 is hollowed out so that it can be held by an index finger and middle finger. As a result, an operator can perform a sliding operation easily using one hand.

Next, a description will be given of a perforation suturing method that sutures the perforation H occurring in a stomach using the clip device 1 having the above described structure.

Firstly, as is shown in FIG 2, a housing step is performed in which the perforation suture clip 3 is housed within the housing sheath 4 with its other end side facing towards the distal end. At this time, the delivery hook 19 is left engaged with the hook portion 11. Next, the insertion portion 2a of the endoscope 2 is introduced through an aperture and guided into the interior of the stomach, and the location of the perforation H is confirmed by endoscopic images. After the location of the perforation H has been confirmed, as is shown in FIG 1, the housing sheath 4 is inserted inside the treatment tool channel 2b of the endoscope 2. Next, the housing sheath 4 is made to protrude from the distal end of the insertion portion 2a while this operation is confirmed by endoscopic image.

Next, a puncturing step is performed in which the rod portion 10 is made to puncture the stomach wall X surrounding the perforation H, and one end side is positioned at the mucous membrane side P 1 of the stomach while the other end side is positioned at the serous membrane side P2 of the stomach. Note that, in the present embodiment, during this puncturing step, as a result of the housing sheath 4 puncturing the stomach directly, the rod portion 10 that is housed in the interior thereof can be positioned in a predetermined location.

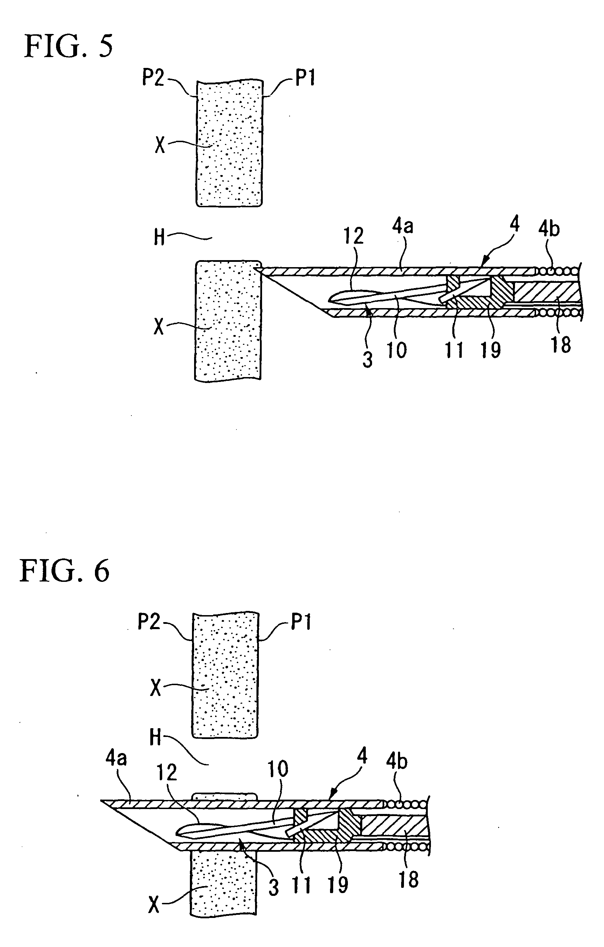

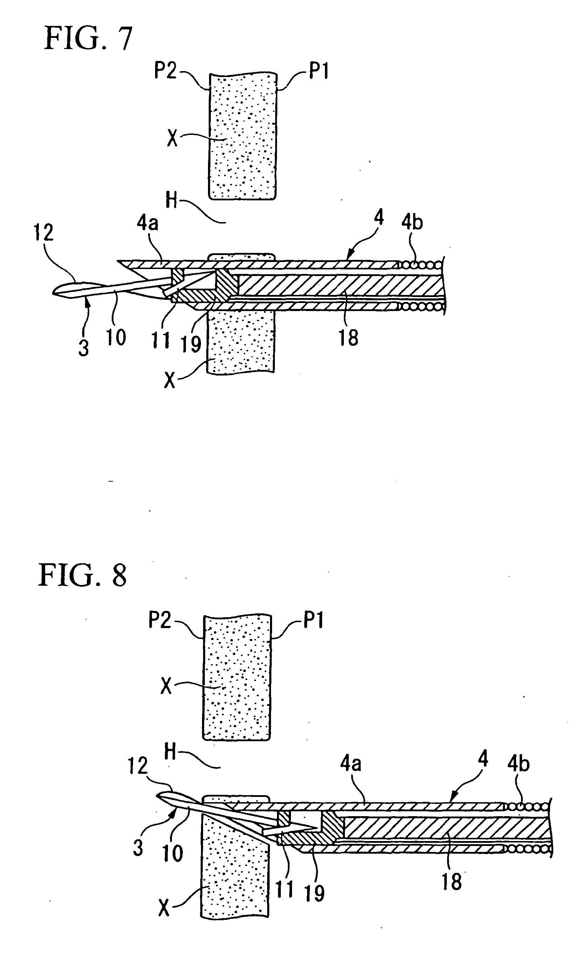

Specifically, as is shown in FIG 5, the needle portion 4a is made to puncture the stomach wall X from the mucous membrane P1 side in the vicinity of the perforation H while the operation is confirmed by endoscopic image. Next, as is shown in FIG. 6, the needle portion 4a is pushed forward so that it penetrates the stomach wall X. After the stomach wall X has been penetrated by the needle portion 4a, as is shown in FIG. 7, the sliding portion 17 is slid along such that the delivery hook 19 is pushed forward to the distal end of the needle portion 4a, and the other end side of the rod portion 10 is made to protrude to the outside from the distal end of the needle portion 4a. Next, as is shown in FIG 8, the entire housing sheath 4 is pulled so as to move slightly into the interior of the stomach. When this is done, because the rod portion 10 that is protruding from the housing sheath 4 is pressed against the stomach wall X, the other end side is in a state of facing up towards the perforation H. Accordingly, it is possible to visually confirm the other end side of the rod portion 10 from the interior of the stomach by endoscopic image through the perforation H.

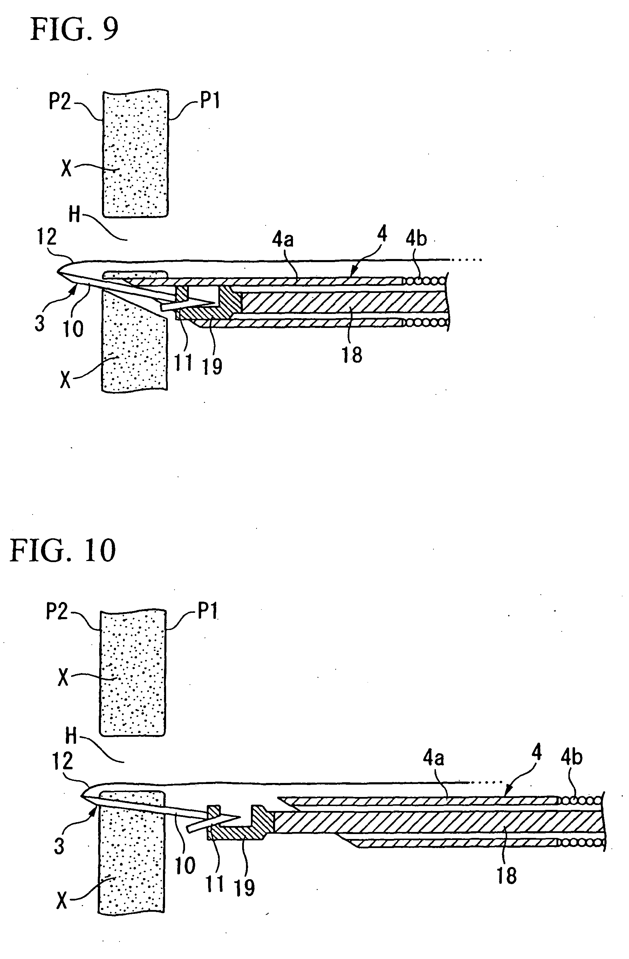

After the other end side of the rod portion 10 has been confirmed, as is shown in FIG. 9, the traction thread 12 that is fixed to the other end side of the rod portion 10 is drawn into the interior of the stomach (i.e., to the mucous membrane side P1) through the perforation H using gripping forceps (not shown) that are inserted into the stomach interior via another treatment tool channel 2b of the endoscope 2. Next, as is shown in FIG. 10, the housing sheath 4 is pulled completely out from the stomach wall X, and the sliding portion 17 is slid along such that the delivery hook 19 is moved slightly to the base end side of the needle portion 4a. As a result, the rod portion 10 can be moved slightly towards the stomach interior side and a small gap can be opened up between the hook portion 11 and the stomach wall X. Moreover, at this time, the other end side of the rod portion 10 is in a state of protruding slightly on the serous membrane side P2. Next, the delivery hook 19 and the hook portion 11 are separated.

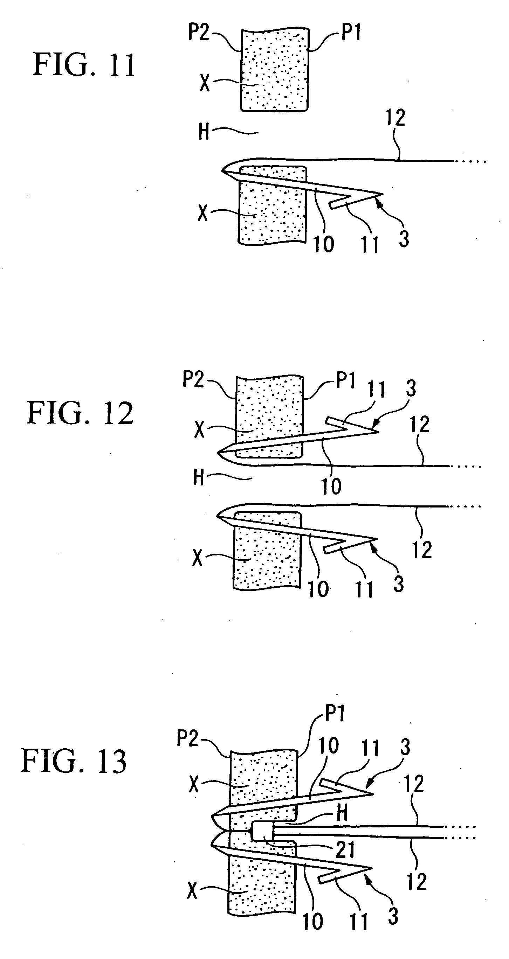

As is shown in FIG 11, the result of this is that the one end side of the rod portion 10 can be positioned on the mucous membrane side P1, while the other end side can be positioned on the serous membrane side P2. Namely, the hook portion 11 is positioned on the mucous membrane side P1 which is the internal side of the stomach, while the other end side to which the traction thread 12 is fixed is positioned on the serous membrane side P2 which is the external side of the stomach. Moreover, as is described above, the traction thread 12 is drawn into the interior of the stomach through the perforation H. At this point, the puncturing step is ended.

Next, the above described puncturing step is repeated, and a rod portion placement step is performed in which another rod portion 10 is made to puncture the stomach wall X peripheral to the perforation H. Note that in the present embodiment, an example is described in which the puncturing step is performed twice and two rod portions 10 are positioned such that they sandwich the perforation H. Accordingly, if this rod portion placement step is performed, as is shown in FIG. 12, two perforation suture clips 3 are in a state of puncturing the stomach wall X.

Next, a traction step is performed in which external force is applied to the two rod portions 10 via the traction threads 12 that are connected to each one so that the other end sides of the rod portions 10 are pulled to the stomach wall X side and the serous membrane side P2 is reefed. Specifically, after the traction threads 12 that have been drawn into the interior of the stomach have been gripped by gripping forceps (not shown), external force is applied by pulling the traction threads 12. In particular, in the present embodiment, as is shown in FIG. 13, the two traction threads 12 are passed through through holes (not shown) in a stopper 21, and the stopper 21 is then moved towards the perforation H thereby pulling the traction threads 21 tight. Consequently, the other end sides of the two rod portions 10 are pulled towards the stomach wall X side, and the perforation H becomes gradually smaller due to the stomach wall X being pulled. As a result, the serous membrane side P2 of the perforation H is reefed and can be closed.

Note that in the present embodiment, the stopper 21 is used, however, it is also possible to achieve the same effect simply by pulling the traction thread 12 without using the stopper 21. However, by using the stopper 21, the serous membrane side P2 can be reefed more effectively.

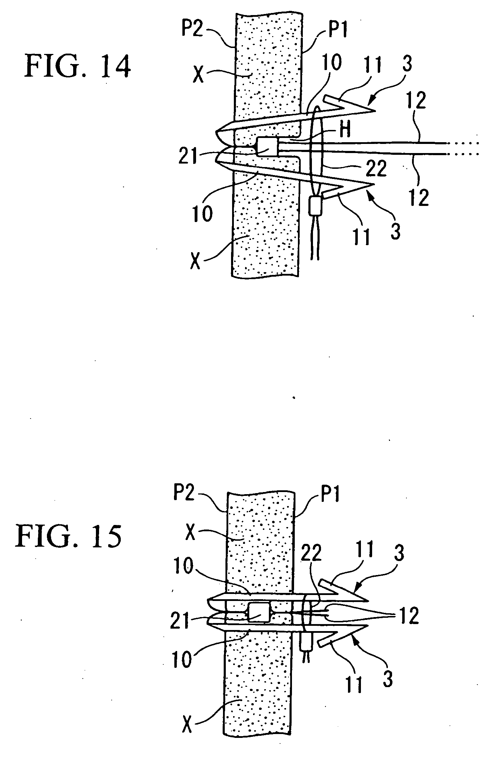

Next, a suturing step is performed in which, while the one end sides of the two rod portions 10 are adjacent to each other, they are joined together via the loop 22, and the perforation H is sutured while the mucous membrane side P1 is in a reefed state. Specifically, as is shown in FIG 14, using gripping forceps or the like the loop 22 is circled around the one end sides of the two rod portions 10 that are positioned on the mucous membrane side P1 of the stomach. By then pulling the circling loop 22 tight, as is shown in FIG 15, the one end sides of the two rod portions 10 are joined together while they are positioned next to each other. Consequently, the mucous membrane side P1 of the stomach can be pulled in the same way. As a result, the mucous membrane side P1 of the perforation H can also be closed in a reefed state. In particular, because the hook portions 11 are bent back at the one end sides of the rod portions 10, the loop 22 is caught on the hook portions 11 and cannot drop off the rod portions 10. Accordingly, this reefed state can be maintained for an extended period of time.

As has been described above, by using the perforation suture clip 3 of the present embodiment, it is possible to reef a perforation H occurring in a stomach from both a serous membrane side P2 and a mucous membrane side P1 and perform reliable suturing without performing an abdominal operation and without opening a hole in a stomach wall. Moreover, unlike a conventional device, because it is possible to close the perforation H on both the internal side and external side of the stomach, the suturing is secure and the reliability of an operation can be improved.

During the traction step, because it is possible to easily apply external force simply by pulling the traction thread 12 that has been drawn into the interior of the stomach, the task of reefing the serous membrane side P2 can be performed easily.

Moreover, according to the clip device 1 of the present embodiment, because the perforation section clip 3 can be housed within the housing sheath 4, the perforation suture clip 3 can approach the vicinity of the perforation H without being brought into contact with any other portions. Accordingly, it is possible to further improve the level of safety of an operation. Moreover, by moving the wire 18 forwards or backwards using the operating section 5, the perforation suture clip 3 that is engaged with the delivery hook 19 can be pushed out from the housing sheath 4, and the engagement between the delivery hook 19 and the hook portion 11 can be eliminated at a desired timing so that the perforation suture clip 3 can be easily detached. In this manner, the perforation suture clip 3 can be handled easily and an improvement in the operability thereof can be achieved.

Furthermore, because the distal end of the housing sheath 4 forms the needle portion 4a, it is not necessary for the perforation suture clip 3 to directly puncture the stomach wall X. Accordingly, in the case of a stomach in which the muscular tunic between the mucous membrane side P1 and the serous membrane side P2 is comparatively strong, it is still possible to reliably leave the perforation suture clip 3 in a predetermined position.

### (Second embodiment)

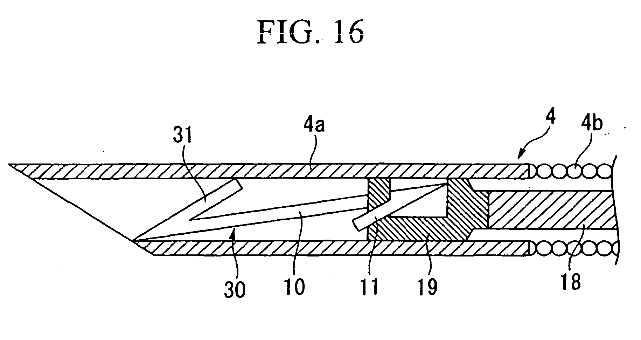

Next, a second embodiment of the perforation suture clip and clip device according to the present invention will be described with reference made to FIG 16 through FIG. 25. Note that portions of the second embodiment that are the same as component elements in the first embodiment are given the same symbols and a description thereof is limited.

The second embodiment differs from the first embodiment in that in the first embodiment the traction thread 12 is fixed to the other end side of the rod portion 10, however, in the second embodiment a folded-back portion (i.e., a traction portion) 31 is formed at the other end side of the rod portion 10.

Namely, as is shown in FIG 16, the perforation suture clip 30 of the present embodiment has a folded-back portion 31 at the other end side of the rod portion 10 that is folded back towards the one end side of the rod portion 10 at a predetermined opening angle. The length of this folded-back portion 31 is adjusted so that the folded-back portion 31 can be drawn into the perforation H when the rod portion 10 punctures the stomach wall X.

Next, a description will be given of a perforation suturing method for suturing a perforation H occurring in a stomach using the perforation suture clip 30 constructed in this manner.

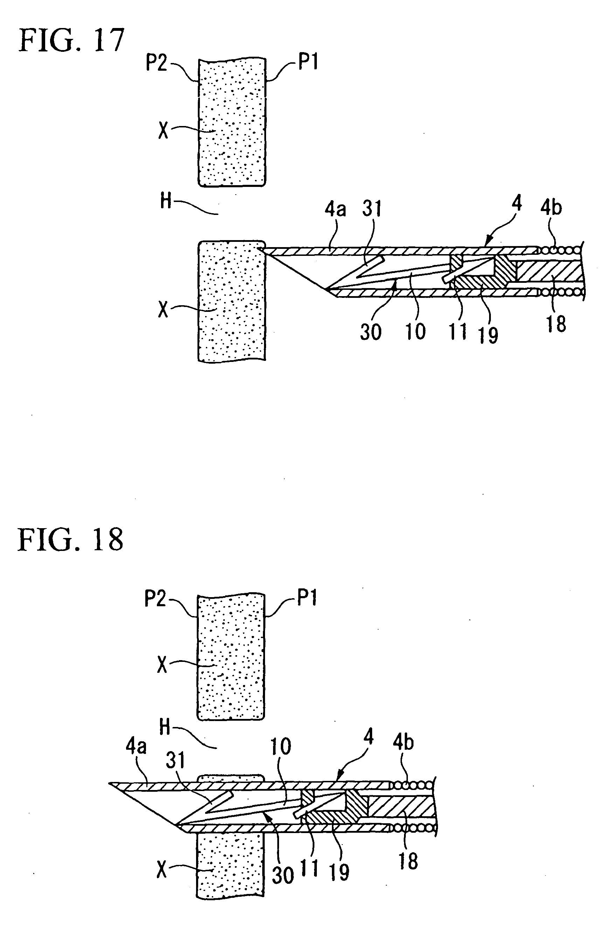

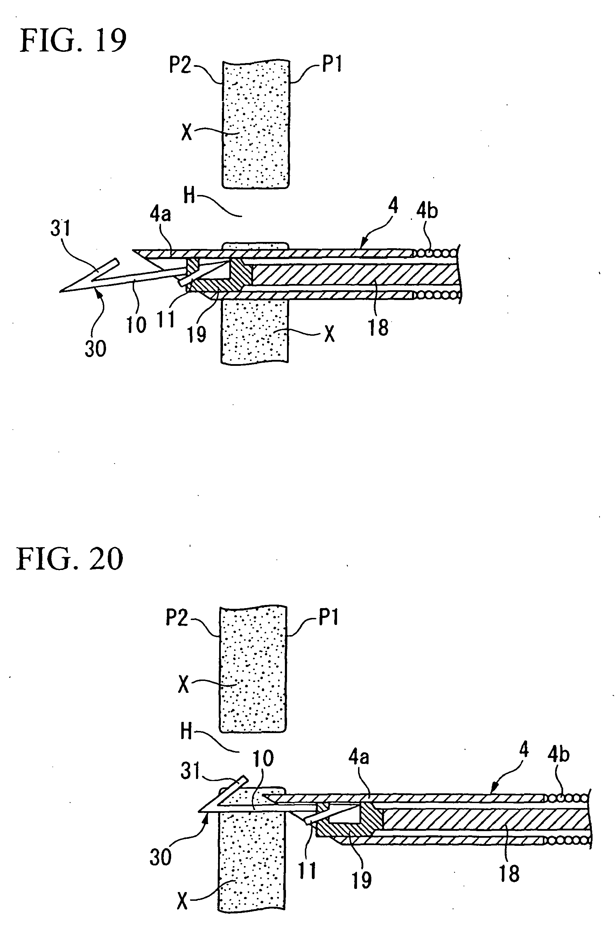

Firstly, in the same way as in the first embodiment, as is shown in FIG 17, the needle portion 4a punctures the stomach wall X from the mucus membrane side P1 in the vicinity of the perforation H and this operation is confirmed using endoscopic images. Next, as is shown in FIG 18, the needle portion 4a is pushed forward so as to penetrate the stomach wall X. After the stomach wall X has been penetrated by the needle portion 4a, as is shown in FIG. 19, the sliding portion 17 is slid along such that the delivery hook 19 is pushed forward to the distal end of the needle portion 4a, and the folded-back portion 31 that is formed at the other end side of the rod portion 10 is made to protrude to the outside from the distal end of the needle portion 4a. Next, as is shown in FIG 20, the entire housing sheath 4 is pulled so as to move slightly into the interior of the stomach. At this time, because the folded-back portion 31 is formed at the other end side of the rod portion 10, this folded-back portion 31 can be drawn into the perforation H so as to catch onto the stomach wall X. Accordingly, the rod portion 10 can be easily positioned at a predetermined location of the stomach wall X. Note that it is possible to visually confirm a portion of the folded-back portion 31 from the interior of the stomach by endoscopic images through the perforation H.

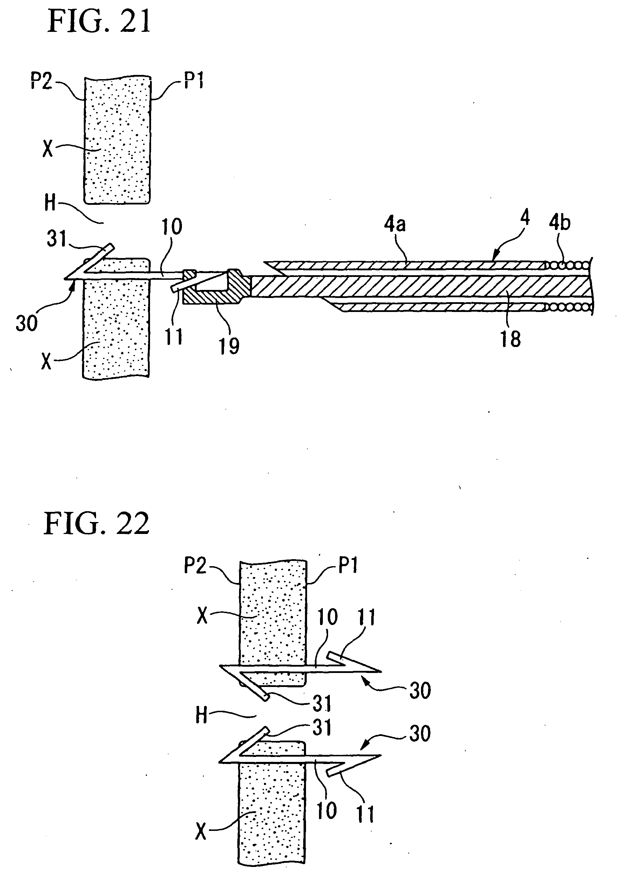

After the folded-back portion 31 has been confirmed, as is shown in FIG 21, the housing sheath 4 is pulled completely out from the stomach wall X, and the sliding portion 17 is slid along such that the delivery hook 19 is moved slightly to the base end side of the needle portion 4a. As a result, the rod portion 10 can be moved slightly towards the stomach interior side and a small gap can be opened up between the hook portion 11 and the stomach wall X. Moreover, the folded-back portion 31 can be reliably engaged with the stomach wall X. Next, the delivery hook 19 and the hook portion 11 are separated.

As a result, the one end side of the rod portion 10 can be positioned on the mucous membrane side P1, while the other end side can be positioned on the serous membrane side P2. Namely, the hook portion 11 is positioned on the mucous membrane side P 1 which is the internal side of the stomach, while the folded-back portion 31 is positioned on the serous membrane side P2 which is the external side of the stomach. At this point, the puncturing step is ended.

Next, the above described puncturing step is repeated so that, as is shown in FIG 22, two perforation suture clips 30 puncture the stomach wall X.

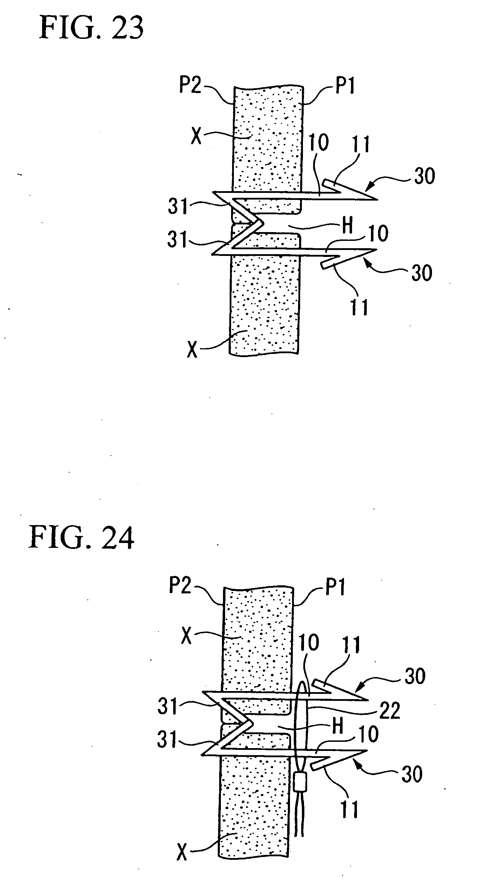

Next, a traction step is performed in which the two rod portions 10 are pulled into the stomach using gripping forceps or the like so that external force is applied via the folded-back portions 31. In particular, because the folded-back portion 31 is drawn into the perforation H so as to become engaged with the stomach wall X, it is easy to apply external force to the stomach wall X. Because of this, the other end side of the rod portion 10 can be pulled to the stomach wall X side easily and reliably. Consequently, the stomach walls X are pulled and the perforation H becomes gradually smaller. As a result, as is shown in FIG 23, the serous membrane side P2 of the perforation H is reefed and can be closed.

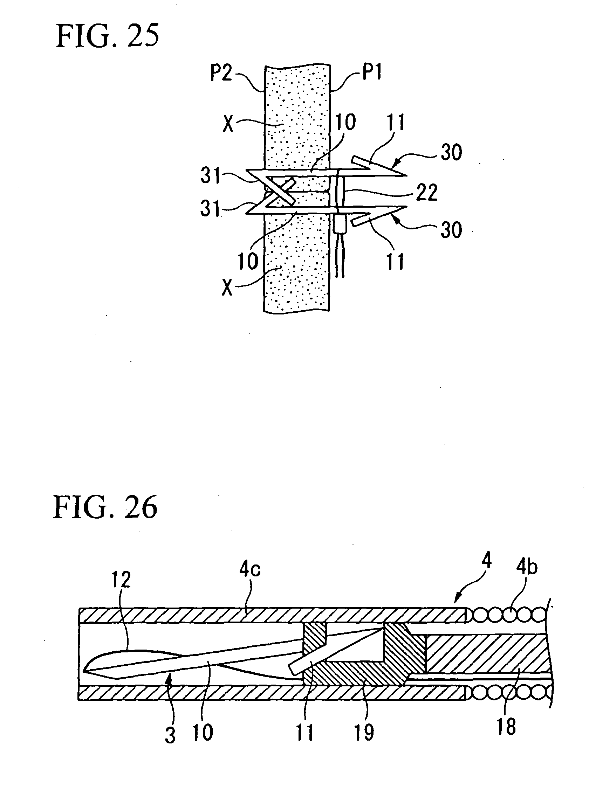

Next, a suturing step is performed in the same way as in the first embodiment. Firstly, as is shown in FIG 24, using gripping forceps or the like the loop 22 is circled around the one end sides of the two rod portions 10 that are positioned on the mucous membrane side P1 of the stomach. By then pulling the circling loop 22 tight, as is shown in FIG. 25, the one end sides of the two rod portions 10 are joined together in a state of being positioned next to each other. As a result, the mucous membrane side P1 of the perforation H can also be closed in a reefed state. In particular, because the hook portions 11 are bent back at the one end sides of the rod portions 10, the loop 22 is caught on the hook portions 11 and cannot drop off the rod portions 10.

In this manner, in the present embodiment as well, in the same way as in the first embodiment, it is possible to reef a perforation H occurring in a stomach from both a serous membrane side P2 and a mucous membrane side P 1 and perform reliable suturing without performing an abdominal operation.

It should be understood that the range of technology of the present invention is not limited to the above described embodiments, and various modifications may be made thereto insofar as they do not depart from the scope of the present invention.

For example, in each of the above described embodiments, an example in which a perforation H has occurred in a stomach is described, however, the present invention is not limited to stomachs and the perforation suture clips 3 and 30 as well as the clip device 1 of the present invention can also be used in cases in which a perforation H has occurred in a digestive tract such as in the small intestine or the large intestine.

Moreover, in each of the above described embodiments, the perforation suture clips 3 and 30 are housed within the housing sheath 4 that has the needle portion 4a whose distal end is formed in a needle shape, however, the present invention is not limited to this and, as is shown in FIG 26, it is also possible for the perforation suture clips 3 and 30 to be housed within a housing sheath 4 that has a cylindrical portion 4c that is formed simply in a circular cylinder shape. Note that in FIG 26 a state in which the perforation suture clip 3 of the first embodiment is housed is shown. In this case, after the perforation suture clip 3 has been made to protrude from the housing sheath 4 in the vicinity of the perforation H, then the stomach wall X may be punctured at the one end side or at the other end side of the sharpened rod portion 10. Namely, it is sufficient if the perforation suture clip 3 directly punctures the stomach wall X. In this case as well, it is possible to achieve the same operational effects.

Moreover, when dragging the other end side of the rod portion 10, the traction thread 12 is used in the first embodiment while the folded-back portion 31 is used in the second embodiment, however, the present invention is not limited to this and any suitable structure may be employed provided that the other end side of the rod portion 10 can be dragged towards the stomach wall X side. Moreover, it is also possible for the traction thread 12 to be fixed to the folded-back portion 31.

When the rod portion 10 punctures the stomach wall X, it is also possible to first move the rod portion 10 to the outer side (i.e., to the serous membrane side P2) of the stomach wall X through the perforation H, and then cause the one end side to puncture the stomach wall X from the serous membrane side P2.

In this case as well, it is possible to achieve the same operational effects.

Moreover, in each of the above described embodiments, only one each of the perforation suture clips 3 and 30 are housed in the housing sheath 4, however, the present invention is not limited to this and it is also possible to house a plurality of perforation suture clips 3 or 30 in a row in the direction of the axis L of the housing sheath 4.

By employing this type of structure, because it is possible to cause the perforation suture clips 3 or 30 to puncture the stomach wall X around a perforation H efficiently and in a short period of time, an improvement in workability can be obtained and a reduction in work time can also be achieved.

Note that the items below are included in the present invention.

### [Supplementary item 1]

A perforation suturing method for suturing a perforation occurring in a digestive tract that uses in combination with an endoscope a perforation suture clip that includes: a rod portion whose two ends have both been sharpened that punctures a digestive tract; a hook portion that is folded back from one end side of the rod portion towards the rod portion side at a predetermined opening angle; and a traction portion that is fixed to another end side of the rod portion and that, when external force is applied thereto, drags the other end side of the rod portion towards the digestive tract side, wherein the perforation suturing method includes:
a puncturing step in which the rod portion is made to puncture the digestive tract around the perforation, and the one end side is positioned on the mucous membrane side of the digestive tract while the other end side is positioned on the serous membrane side of the digestive tract;
a rod portion placement step in which the puncturing step is repeated a plurality of times so that a plurality of the rod portions are made to puncture the digestive tract around the perforation;
a traction step in which external force is applied via a plurality of the traction portions, and the other end sides of the plurality of rod portions are dragged towards the digestive tract thereby reefing the serous membrane side; and
a suturing step in which, while the one end sides of the plurality of rod portions are adjacent to each other, they are joined together via a wire material, and the perforation is sutured while the mucous membrane side is in a reefed state.

### [Supplementary item 2]

The perforation suturing method according to claim 1, wherein
during the puncturing step, the other end side of the rod portion is made to puncture from the mucous membrane side.

### [Supplementary item 3]

The perforation suturing method according to claim 1, wherein
during the puncturing step, the rod portion is first moved outside the digestive tract through the perforation, and the one end side is then made to puncture the digestive tract from the serous membrane side.

### [Supplementary item 4]

The perforation suturing method according to any one of clams 1 to 3, wherein
the traction portion is a folded-back portion that is folded back at a predetermined opening angle towards the rod portion side, and
during the puncturing step, the rod portion is made to puncture the digestive tract such that the folded-back portion is drawn into the perforation.

### [Supplementary item 5]

The perforation suturing method according to any one of claims 1 to 3, wherein
the traction portion is a traction thread having a predetermined tensile strength, and
during the puncturing step, the traction thread is drawn through the perforation to the mucous membrane side.

### [Supplementary item 6]

The perforation suturing method according to any one of clams 1 to 5, wherein
prior to the puncturing step, a housing step is performed in which the rod portion is housed, with the other end side facing towards the distal end, within a housing tool whose distal end is formed in a needle shape and that is inserted inside a channel in an endoscope, and
during the puncturing step, the rod portion is placed in a predetermined position as a result of the housing tool puncturing the digestive tract.

## Claims

1. A perforation suture clip (3, 30) that is used in combination with an endoscope (2) and sutures a perforation (H) occurring in a digestive tract, **characterized in that**:
the perforation suture clip (3, 30) comprises
a rod portion (10) whose two ends are sharpened;
a hook portion (11) that is bent back from one end side of the rod portion (10) at a predetermined opening angle towards the other end side of the rod portion (10); and
a traction portion (12, 31) that is fixed to the other end side of the rod portion (10) and drags the other end side of the rod portion (10).

2. The perforation suture clip (30) according to claim 1, **characterized in that**:
the traction portion (31) is a folded-back portion that can be folded back at a predetermined opening angle towards the one end side of the rod portion (10), and can be drawn into the perforation (H).

3. The perforation suture clip (3) according to claim 1, **characterized in that**:
the traction portion (12) is a traction thread that has a predetermined tensile strength, and can be drawn through the perforation (H) to a mucous membrane side (P1).

4. A clip device (1) that is used in combination with an endoscope (2) and sutures a perforation (H) occurring in a digestive tract, **characterized in that**:
the clip device (1) comprises
the perforation suture clip (3, 30) according to claim 1;
a housing tool (4) that can be inserted into a channel (2b) of the endoscope (2), and whose interior houses the rod portion (10) with the other end side facing towards a distal end of the housing tool (4);
an engaging portion (19) that engages with the hook portion (11);
an operation transmitting device (18) that is connected to the engaging portion (19), and is able to move backwards and forwards freely relative to the housing tool (4); and
an operating section (5) that is connected to the operation transmitting device (18), and moves the housed rod portion (10) backwards and forwards in the axial direction of the housing tool (4) by moving the operation transmitting device (18) backwards and forwards, and that terminates the engagement between the engaging portion (19) and the hook portion (11) by pushing the rod portion (10) out from the housing tool (4).

5. The clip device (1) according to claim 4, **characterized in that**:
a plurality of the perforation suture clips (3, 30) are housed in the housing tool (4) by being lined up in a row in the axial direction.

6. The clip device (1) according to claim 4, **characterized in that**:
the distal end of the housing tool (4) is formed in a needle shape that enables the housing tool (4) to puncture the digestive tract.

## Patentansprüche

1. Perforationsnahtklemme (3, 30), die in Verbindung mit einem Endoskop (2) verwendet wird und eine in einem Verdauungstrakt auftretende Perforation (H) zunäht, **dadurch gekennzeichnet, dass**:
die Perforationsnahtklemme (3, 30) aufweist
- ein Stabteil (10), dessen zwei Enden spitz sind;
- ein Hakenteil (11), das mit einem vorbestimmten Öffnungswinkel von einer Endseite des Stabteils (10) zu der anderen Endseite des Stabteils (10) zurückgebogen ist; und
- ein Zugteil (12, 31), das an der anderen Endseite des Stabteils (10) angebracht ist und die andere Endseite des Stabteils (10) zieht.

2. Perforationsnahtklemme (30) nach Anspruch 1, **dadurch gekennzeichnet, dass**:
das Zugteil (31) ein nach hinten abgekantetes Teil ist, das mit einem vorbestimmten Öffnungswinkel zu der einen Endseite des Stabteils (10) zurückgefaltet, und in die Perforation (H) gezogen werden kann.

3. Perforationsnahtklemme (3) nach Anspruch 1, **dadurch gekennzeichnet, dass**:
das Zugteil (12) ein Zugfaden mit einer vorbestimmten Dehnbarkeit ist, und durch die Perforation (H) zu einer Schleimhautseite (P1) gezogen werden kann.

4. Klemmvorrichtung (1), die in Verbindung mit einem Endoskop (2) verwendet wird und eine in einem Verdauungstrakt auftretende Perforation zunäht, **dadurch gekennzeichnet, dass**:
die Klemmvorrichtung (1) aufweist
- die Perforationsnahtklemme (3, 30) nach Anspruch 1;,
- ein Aufnahmewerkzeug (4), das in einen Kanal (2b) des Endoskops (2) eingeführt werden kann, und dessen Innenraum das Stabteil (10) aufnimmt, wobei die andere Endseite zu einem Distalende des Aufnahmewerkzeugs (4) gewandt ist;
- ein Verbindungsteil (19), das mit dem Hakenteil (11) in Eingriff tritt;
- eine Betätigungsübertragungsvorrichtung (18), die mit dem Verbindungsteil (19) verbunden ist, und sich relativ zu dem Aufnahmewerkzeug (4) frei nach hinten und nach vorne bewegen kann; und
- einen Betätigungsabschnitt (5), der mit der Betätigungsübertragungsvorrichtung (18) verbunden ist und durch Rückwärts- und Vorwärtsbewegung der Betätigungsübertragungsvorrichtung (18) das aufgenommene Stabteil (10) in die Axialrichtung des Aufnahmewerkzeugs (4) rückwärts und vorwärts bewegt, und der durch Drücken des Stabteils (10) aus dem Aufnahmewerkzeug (4) die In-Eingriffbringung zwischen dem Verbindungsteil (19) und dem Hakenteil (11) beendet.

5. Klemmvorrichtung (1) nach Anspruch 4, **dadurch gekennzeichnet, dass**:
durch Anordnung in einer Reihe in der Axialrichtung eine Mehrzahl von Perforationsnahtklemmen (3, 30) in dem Aufnahmewerkzeug (4) aufgenommen sind.

6. Klemmvorrichtung (1) nach Anspruch 4, **dadurch gekennzeichnet, dass**:
das Distalende des Aufnahmewerkzeugs (4) in einer Nadelform gebildet ist, die dem Aufnahmewerkzeug (4) ermöglicht, den Verdauungstrakt zu durchstechen.

## Revendications

1. Agrafe pour suture de perforations (3, 30) qui est utilisée en combinaison avec un endoscope (2) et suture une perforation (H) dans un tube digestif, **caractérisée en ce que** :
l'agrafe pour suture de perforations (3, 30) comprend
une partie de tige (10) dont les deux extrémités sont aiguisées ;
une partie de crochet (11) qui est repliée vers l'arrière à partir d'un côté d'extrémité de la partie de tige (10) à un angle d'ouverture prédéterminé vers l'autre côté d'extrémité de la partie de tige (10) ; et
une partie de traction (12, 31) qui est fixée à l'autre côté d'extrémité de la partie de tige (10) et tire l'autre côté d'extrémité de la partie de tige (10).

2. Agrafe pour suture de perforations (30) selon la revendication 1, **caractérisée en ce que** :
la partie de traction (31) est une partie repliée qui peut être repliée à un angle d'ouverture prédéterminé vers l'un côté d'extrémité de la partie de tige (10), et peut être tirée à l'intérieur de la perforation (H).

3. Agrafe pour suture de perforations (3) selon la revendication 1, **caractérisée en ce que** :
la partie de traction (12) est un fil de traction qui a une résistance à la traction prédéterminée, et peut être tiré à l'intérieur de la perforation (H) jusqu'à un côté de membrane muqueuse (P1).

4. Dispositif d'agrafe (1) qui est utilisé en combinaison avec un endoscope (2) et suture une perforation (H) dans un tube digestif, **caractérisé en ce que** :
le dispositif d'agrafe (1) comprend
l'agrafe pour suture de perforations (3, 30) selon la revendication 1 ;
un outil de logement (4) qui peut être inséré dans un canal (2b) de l'endoscope (2), et dont l'intérieur reçoit la partie de tige (10) avec l'autre côté d'extrémité faisant face vers une extrémité distale de l'outil de logement (4) ;
une partie d'engagement (19) qui s'engage avec la partie de crochet (11) ;
un dispositif de transmission de fonctionnement (18) qui est connecté à la partie d'engagement (19), et est apte à se déplacer vers l'arrière et vers l'avant librement par rapport à l'outil de logement (4) ; et
une section opérationnelle (5) qui est connectée au dispositif de transmission de fonctionnement (18), et déplace la partie de tige (10) reçue vers l'arrière et vers l'avant dans le sens axial de l'outil de logement (4) en déplaçant le dispositif de transmission de fonctionnement (18) vers l'arrière et vers l'avant, et qui met fin à l'engagement entre la partie d'engagement (19) et la partie de crochet (11) en faisant sortir par poussée la partie de tige (10) de l'outil de logement (4).

5. Dispositif d'agrafe (1) selon la revendication 4, **caractérisé en ce que** :
une pluralité des agrafes pour suture de perforations (3, 30) sont reçues dans l'outil de logement (4) en étant alignées en une rangée dans le sens axial.

6. Dispositif d'agrafe (1) selon la revendication 4, **caractérisé en ce que** :
l'extrémité distale de l'outil de logement (4) est formé à une forme d'aiguille qui permet que l'outil de logement (4) perce le tube digestif.
